(19) **Europäisches Patentamt**

**European Patent Office**

**Office européen des brevets**

(11) Publication number: **0 358 324**

**A1**

(12) # EUROPEAN PATENT APPLICATION

(21) Application number: **89307813.9**

(51) Int. Cl.5: **A61F 2/08**

(22) Date of filing: **01.08.89**

(30) Priority: **05.08.88 GB 8818614**

(43) Date of publication of application:
**14.03.90 Bulletin 90/11**

(84) Designated Contracting States:
**DE FR GB IT**

(71) Applicant: **ELLIS DEVELOPMENTS LIMITED**
**68 Carlton Road**
**Nottingham NG3 2AP(GB)**

(72) Inventor: **Ellis, Julian Garth**
**18 Tavistock Avenue Mapperley Park**
**Nottingham, NG3 5BD(GB)**

(74) Representative: **Low, Peter John et al**
**WILSON, GUNN & ELLIS 41 Royal Exchange**
**Manchester, M2 7BD(GB)**

(54) **Prosthetic ligament.**

(57) A Y-shaped prosthetic ligament that can be used to replace two knee ligaments and which comprises a main prosthesis formed by a length of fabric having a pocket (14) and a tubular part (12) and an integral second prosthesis (30) which extends from the main prosthesis intermediate the ends thereof.

FIG.1

# A PROSTHETIC LIGAMENT

This invention relates to a prosthetic ligament.

It is often the case that the medial collateral ligament of a knee is damaged or ruptured. However, such an injury is not normally sufficiently serious in its effect to warrant any repair on its own. However, a knee injury which damages the anterior cruciate ligament normally requires repair by means of a prosthetic ligament. The repair or replacement of the anterior cruciate ligament provides a good opportunity for also repairing or replacing the lateral collateral ligament or the medial collateral ligament during the same operation. The anterior cruciate ligament can be replaced by a prosthetic ligament of the kind described in European Patent No.0126520. For repair or replacement of lateral collateral ligament or the medial collateral ligament a separate natural or synthetic prosthetic device is used or a graft is taken from some other part of the patient's body.

One of the major difficulties of ligament repair or replacement is in the anchoring of the prosthesis securely within the body. This has in the past been carried out by screws, staples, toggles and similar methods. A bone-plug anchoring technique has been described in European Patent Application 0126520. This technique has proved to be very successful, and is very widely used.

The technique of bone plug anchoring has been applied to replacements of the anterior cruciate ligaments of the knee, and also to the posterior cruciate ligaments. The same technique has also been used in the repair of other joints and ligament repair or replacement, but there has been no method of making a prosthesis which can replace the two ligaments during the same operation conveniently.

The object of the present invention is the provision of a prosthetic ligament which can be used for simultaneous replacement of two ligaments and more particularly pairs of ligaments such as the medial collateral and posterior cruciate ligaments in the knee or ligaments in the shoulder.

According to the present invention there is provided a prosthetic ligament comprising a main prosthesis, said main prosthesis comprising a length of fabric formed as a tube over at least a part of said length, a pocket adjacent one end of the tubular part of said main prosthesis and a tail extending from the other end of the tubular part, a second prosthesis integral with the main prosthesis, said second prosthesis comprising a length of fabric extending from the main prosthesis intermediate the ends of the main prosthesis.

In one embodiment of the invention the prosthetic ligament of the invention is made by weaving for example in a leno or mock leno weave. The main prosthesis can be made in substantially the same way as is described in US Patent Specification No.4665951 and preferably has from 10 to 50 apertures per cm², preferably from 15 to 36 apertures per cm². The second prosthesis is also woven and is preferably produced from a plurality of additional warp ends provided in the main prosthesis. The woven structure of the second prosthesis can be the same or different to that of the main prosthesis. Preferably the second prosthesis is a leno or mock leno weave with an aperture density within the preferred range of the main prosthesis.

Specific embodiments of the invention will now be described by way of example with reference to the accompanying drawings in which:-

Fig.1 shows a prosthetic ligament in perspective side elevation;

Fig.2 shows diagrammatically the use of the prosthetic ligament of Fig.1 for the repair of knee ligaments;

Fig 3. shows another embodiment of a prosthetic ligament in perspective and side elevation; and

Fig.4 illustrates diagrammatically the use of the prosthesis of Fig.3 in the repair of knee ligaments.

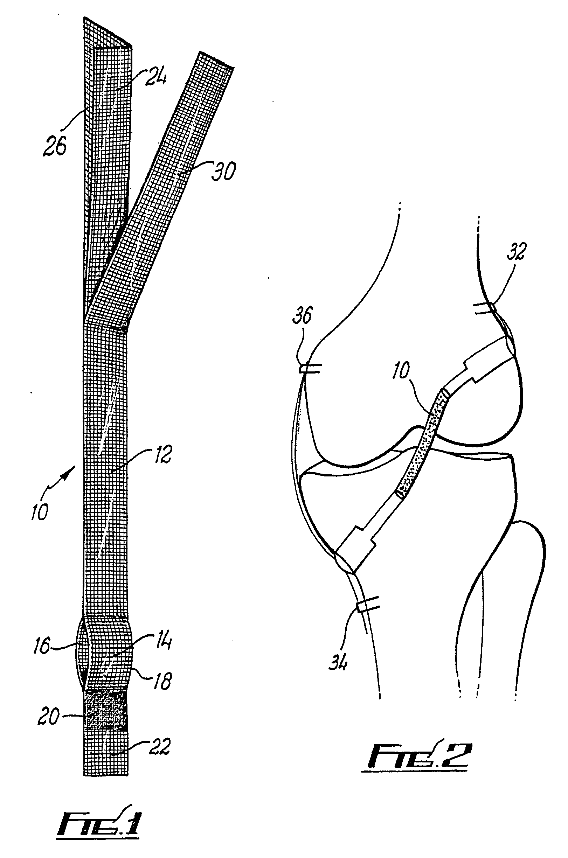

Referring to Fig.1 of the drawings a prosthetic ligament comprises a main prosthesis 10 which has a structure similar to that of the prosthetic ligament described in U.S. Patent No.4665951. Thus the main prosthesis comprises a woven tubular portion 12 having a tail at one end which comprises a pocket 14 which is open at one side 16 and closed at the other 18. Adjacent the pocket 14 on the side remote from tubular portion 12 there is a tightly woven portion 20 and that end of the prosthesis terminates in a terminal portion 22 which may be tubular like portion 12 but need not necessarily be so. The other end of the prosthesis is in the form of a pocket-like tail 24 which has the same overall dimensions as tubular portion but is longitudinally open along its whole length as at 26.

The main prosthesis may be woven in the same way as described in the aforesaid U.S. Patent No.4665951 except that an additional set of warp yarns is provided which are woven into the main prosthesis through portions 22, 20, 14 and 12. At the point where the tubular portion ends and the tail 24 begins the additional warp ends are separated from the other warp yarns and used to form an integral, woven side branch 30 which constitutes the second prosthesis. In the formation of tail 24 and side branch 30 a small number of picks, for example about twelve for each part are preferably alternately woven using a separate shuttle for each part. It will be understood, however, that the side branch 30 can be formed in other ways.

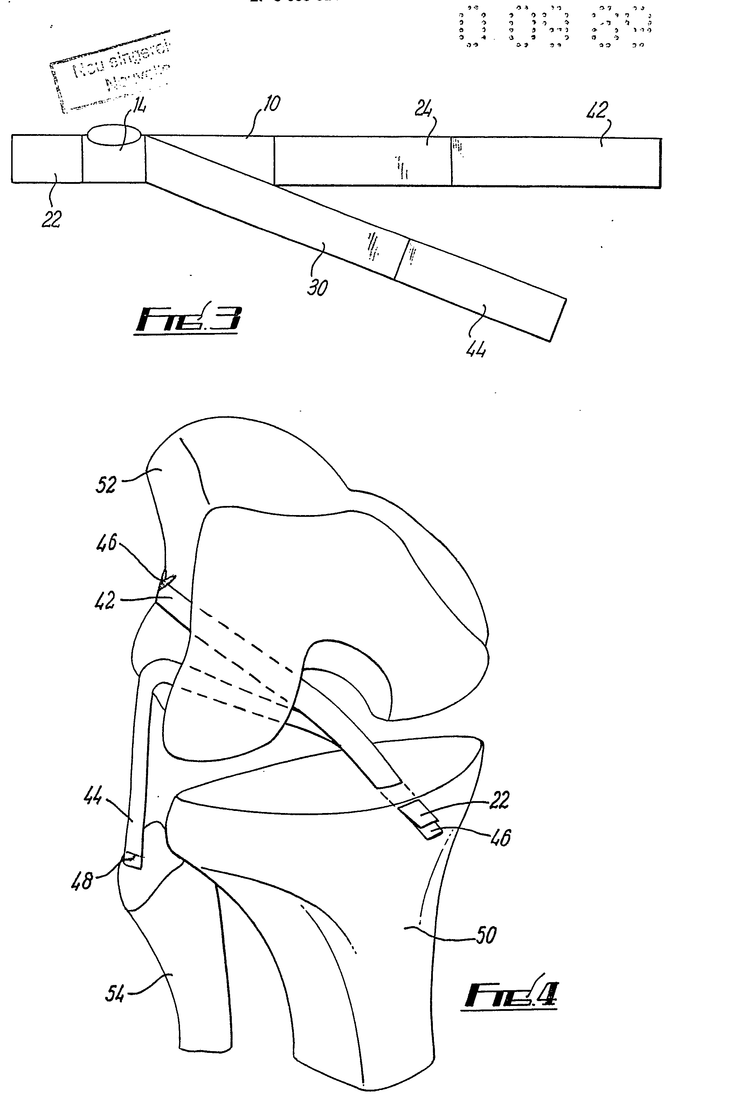

Fig.2 illustrates one way of using the prosthesis of Fig.1. The main prosthesis 10 is used to replace the anterior cruciate ligament in a known manner. The main prosthesis is plugged adjacent each end and then the tails 24 and 20 are stapled to the femur as at 32 and to the tibia as at 34 respectively. The side branch 30 is thus conveniently in position to replace the medial collateral ligament. One end for the medial collateral prosthesis is already fixed by virtue of the fixing of the main prosthesis so that all that is necessary is to staple the free end of the second prosthesis to the femur as indicated at 36.

In the embodiment of Fig.3 the side branch 30 extends from the main prosthesis from between the pocket 14 and the tubular portion. In addition tails 42 and 44 are provided on the free ends of the main and second prosthesis respectively. The tails are preferably of denser weave than the mock leno weave of the prostheses, for example a plain weave or a twill.

A convenient way of using the prosthesis of Fig.3 is illustrated in Fig.4. In this instance the main prosthesis is being used to replace the anterior curicate ligament and the lateral collateral ligament although it will be appreciated that the prosthesis could also be used to replace other pairs of ligaments such as the posterior cruciate ligament together with the medial collateral ligament or the anterior cruciate ligament together with either the lateral collateral ligament or the medial collateral ligament.

A part of the semi-tendinosus is freed from beside the knee joint and is inserted through the pocket 14, and into the tubular part 12 of the main prosthesis and loosely secured thereto by sewing. The prosthesis and tendon are together inserted through a bore in the tibia 50. The rest of the main prosthesis is led through a bore in the femur 52 so that the main prosthesis takes up the place of the anterior cruciate ligament. The tails 22 and 42 of the main prosthesis are fixed as by staples 46 or other fixing means. The second prosthesis is led through a second bore in the femur and then down to the fibula 54 to replace the lateral collateral ligament. The tail 44 of the second prosthesis is fixed to the fibula 54 as by a staple 48 after appropriate tensioning.

The present invention, therefore, provides a simple prosthesis which enables two ligaments to be replaced simultaneously with the relative positions of the ligaments being readily fixed and with the minimum amount of stapling or other fixation.

The invention is not restricted to the described specific embodiment and many modifications and variations can be made.

## Claims

1. A prosthetic ligament comprising a main prosthesis, said main prosthesis comprising a length of fabric formed as a tube over at least a part of said length, a pocket adjacent one end of the tubular part of said main prosthesis and a tail extending from the other end of the tubular part, a second prosthesis integral with the main prosthesis, said second prosthesis comprising a length of fabric extending from the main prosthesis intermediate the ends of the main prosthesis.

2. A prosthetic ligament as claimed in Claim 1, wherein the second prosthesis extends from the main prosthesis adjacent the other end of the tubular part.

3. A prosthetic ligament as claimed in Claim 1, wherein the second prosthesis extends from the main prosthesis adjacent the one end of the tubular part.

4. A prosthetic ligament as claimed in Claim 3, wherein the second prosthesis extends from the main prosthesis intermediate the pocket and the tubular part.

5. A prosthetic ligament as claimed in any preceding claim, wherein a tail is provided at one or more free end of the main prosthesis and/or the second prosthesis.

6. A prosthetic ligament as claimed in Claim 5, wherein at least a part of the or each tail is denser than the remainder of the prosthesis.

7. A prosthetic ligament as claimed in any preceding claim, wherein said main prosthesis and/or the second prosthesis is at least in part an open weave structure.

8. A prosthetic ligament as claimed in Claim 7, wherein the second prosthesis is constructed using warp ends that are also woven in the main prosthesis.

FIG.1

FIG.2

FIG.3

FIG.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | US-A-4 665 951 (J. G. ELLIS) <br> * figure 1; claim 1 * | 1 | A 61 F 2/08 |
| A | | 7 | |
| Y | EP-A-0 236 821 (GEBRUEDER SULZER AG) <br> * figures 1,3; claim 1 * | 1 | |
| A | | 4,5,7,8 | |
| A | EP-A-0 169 045 (JOHNSON & JOHNSON) <br> * page 4, lines 31-35; figure 7 * | 1 | |
| A | EP-A-0 145 492 (SUGICRAFT) <br> * figure 7 * | 1,3,5 | |
| A | FR-A-2 592 578 (P. FREMIGACCI) <br> * figure 3 * | 1,3,5 | |
| D,A | EP-A-0 126 520 (B. SEEDHOM et al.) <br> * figures 1,9,10 * | 1 | |

TECHNICAL FIELDS
SEARCHED (Int. Cl.5)

A 61 F

The present search report has been drawn up for all claims

| Place of search | Date of completion of the search | Examiner |
|---|---|---|
| BERLIN | 21-11-1989 | KANAL P K |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)